(19) Europäisches Patentamt
European Patent Office
Office européen des brevets

(11) Veröffentlichungsnummer: **0 312 921 B1**

(12) **EUROPÄISCHE PATENTSCHRIFT**

(45) Veröffentlichungstag der Patentschrift: **23.06.93**

(51) Int. Cl.⁵: **C07C 69/145**, C07C 67/297, C07C 69/07

(21) Anmeldenummer: **88117079.9**

(22) Anmeldetag: **14.10.88**

# (54) Verfahren zur Herstellung ungesättigter Monoester.

(30) Priorität: **22.10.87 DE 3735755**

(43) Veröffentlichungstag der Anmeldung:
**26.04.89 Patentblatt 89/17**

(45) Bekanntmachung des Hinweises auf die Patenterteilung:
**23.06.93 Patentblatt 93/25**

(84) Benannte Vertragsstaaten:
**BE CH DE ES FR GB IT LI NL**

(56) Entgegenhaltungen:
**DE-A- 2 732 075**
**US-A- 3 804 887**

(73) Patentinhaber: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18 c**
**W-6710 Frankenthal(DE)**
Erfinder: **Siegel, Hardo, Dr.**
**Hans-Purrmann-Allee 25**
**W-6720 Speyer(DE)**

**EP 0 312 921 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung ungesättigter Monoester durch Abspaltung einer Carbonsäure aus Diestern in Gegenwart von Katalysatoren.

Es ist bekannt, daß die Pyrolyse von acetoxylierten 1,4-Butandiolen bei 525 °C zu Butadien führt, aber die Reaktion nicht beim ungesättigten Monoacetat stehen bleibt (J. Polym. Sci. Polym. Chem. 18 (1980) 1729-1758). In BE 766 147 wird mitgeteilt, daß ungesättigte Acetate durch pyrolytische Abspaltung einer Carbonsäure aus Glykoldiacetaten hergestellt werden können, jedoch verläuft diese Pyrolyse unselektiv.

Es wurde gefunden, daß man ungesättigte Monoester der Formel (I)

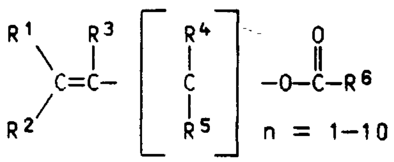

(I)

herstellt, indem man Diester der Formel (II)

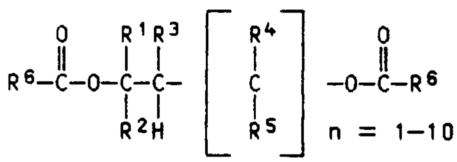

(II)

wobei die Reste $R^1$ bis $R^5$ in den Formeln (I) und (II) einander gleich sind und für den Wasserstoff, geradkettige oder verzweigte Alkyl- oder Alkenylreste mit 1 bis 10 Kohlenstoffatomen, Cycloalkyl- oder Cycloalkylenreste mit 5 bis 8 Kohlenstoffatomen, Aryl-, Alyklaryl- oder Aralkylreste stehen, unter Abspaltung von Carbonsäuren $R^6COOH$, wobei $R^6$ für Wasserstoff oder Alkylrest mit 1 bis 6 Kohlenstoffatomen steht, in Gegenwart von Zeolithen und/oder Phosphaten als Katalysatoren umsetzt. n bedeutet eine ganze Zahl von 1 bis 10.

Als Rest $R^1$ bis $R^6$ kommen Wasserstoff sowie geradkettige oder verzweigte Alkyl- oder Alkenylreste mit 1 bis 10, vorzugsweise 1 bis 4 Kohlenstoffatomen in Betracht.

Alkylreste sind z.B. Methyl-, Ethyl-, Propyl-, n-Butyl-, i-Butyl-, Pentyl-, Hexyl-, Octyl- oder Decylreste. Alkenylreste sind z.B. Propenyl-, Butenyl-, Hexenyl- oder Octenylreste.

Als Cycloalkylreste für $R^1$ bis $R^5$ kommen z.B. Cyclopentyl-, Cyclohexyl-, Cycloheptyl-, Cyclopentenyl- oder Cyclohexenylreste in Betracht.

Aromatische Reste für $R^1$ bis $R^5$ sind z.,B. Phenyl-, Benzyl-, Toluyl-, Phenyl-ethyl-, p-Methylbenzyl- oder p-Propylphenylreste.

Als Rest $R^6$ kommen z.B. Wasserstoff-, Ethyl-, Propyl-, Butyl-, Hexylreste in Betracht.

Als Katalysatoren für das erfindungsgemäße Verfahren verwendet man Zeolithe und/oder Phosphate. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2 (siehe Ullmanns Encyclopädie d. techn. Chemie, 4. Auflage, Band 24, Seite 575 (1983)). Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

Die Zeolithe werden zumeist in der aciden Form angewendet. In den Zeolithen können anstelle von Aluminium auch andere Elemente, wie B, Ga, Fe, Cr, V, As, Sb, Bi oder Be oder deren Gemische in das Gitter eingebaut werden, oder das Silicium kann durch ein anderes vierwertiges Element, wie Ge, Ti, Zr, Hf ersetzt werden.

Entsprechend ihrer Struktur werden Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder

Y.

Für das erfindungsgemäße Verfahren in Betracht kommende Katalysatoren sind Zeolithe aus der Mordenit-Gruppe oder engporige Zeolithe vom Erionit-bzw. Chabasit-Typ oder Zeolithe vom Faujasit-Typ, z.B. Y-, X- oder L-Zeolithe. In diese Gruppen von Zeolithen gehören auch die sogenannten "ultrastabile" Zeolithe des Faujasittyps, d.h. dealuminierte Zeolithe. Verfahren zur Herstellung solcher Zeolithe sind beschrieben in "Catalysis by Zeolites" Band 5 aus "Studies in Surface Science and Catalysis" ed. B. Imelik et al. Elsevier Scientific Publishing Comp. 1980, S. 203 und "Crystal Structures of Ultra-stable Faujasites" Advances in Chemistry Series Nr. 101, American Chemical Society Washington, DC, S. 226 ff (1971) und in US-PS 4 512 961.

Besonders vorteilhaft verwendet man Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet sowie durch Porengrößen, die zwischen denen der Zeolithe vom Typ A und denen vom Typ X oder Y liegen.

Diese Zeolithe können verschiedene chemische Zusammensetzungen aufweisen. Es handelt sich hierbei um Alumino-, Boro-, Eisen-, Beryllium-, Gallium-, Chrom-, Arsen-, Antimon- und Wismutsilikatzeolithode oder deren Gemische sowie Alumino-, Boro-, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen, wie 1,6-Hexandiamin- oder 1,3-Propandiamin- oder Triethylentetramin-Lösung mit oder insbesondere ohne Alkali- oder Erdalkalizusatz bei 100 bis 220 °C unter autogenem Druck hergestellt. Hierzu gehören auch die isotaktischen Zeolithe nach EP 34 727 und EP 46 504. Die erhaltenen Aluminosilikatzeolithe weisen je nach Wahl der Ausgangsstoffmengen ein $SiO_2/Al_2O_3$-Verhältnis von 10 bis 40 000 auf. Derartige Aluminosilikatzeolithe kann man auch in etherischem Medium, wie Diethylenglykoldimethylether, in alkoholischem Medium, wie Methanol bzw. 1,4-Butandiol oder in Wasser synthetisieren.

Borosilikatzeolithe werden z.B. bei 90 bis 200 °C unter autogenem Druck synthetisiert, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin,-, 1,3-Propandiamin- oder Triethylentetraminlösung mit und insbesondere ohne Alkali- oder Erdalkalizusatz zur Reaktion bringt. Hierzu gehören auch die isotaktischen Zeolithe nach EP 34 727 und EP 46 504. Solche Borosilikatzeolithe erhält man auch, wenn man die Reaktion statt in wäßriger Aminlösung in etherischer Lösung, z.B. Diethylenglykoldimethylether oder in alkoholischer Lösung, z.B. in 1,6-Hexandiol durchführt.

Eisensilikatzeolithe erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220 °C unter autogenem Druck.

Zu den verwendbaren siliciumreichen Zeolithen ($SiO_2/Al_2O_3 \geqq 10$) gehören auch die sog. ZSM-Typen, Ferrierit, NU-1 und Silicalit®, ein Molekularsieb, ein sog. Silica Polymorph.

Die so hergestellten Alumino-, Boro- und Eisensilikatzeolithe können nach ihrer Isolierung, Trocknung bei 100 bis 160 °C, vorzugsweise 110 °C und Calcinierung bei 450 bis 550 °C, vorzugsweise 500 °C , mit einem Bindemittel im Verhältnis 90:10 bis 40:60 Gew.% zu Strängen oder Tabletten verformt werden. Als Bindemittel eignen sich verschiedene Aluminiumoxide, insbesondere Boehmit, amorphe Aluminosilikate mit einem $SiO_2/Al_2O_3$-Verhältnis von 25:75 bis 90:5, insbesondere 75:25, Siliciumdioxid, insbesondere hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate der Preßlinge bei 110 °C/16 h getrocknet und bei 500 °C/16 h calciniert.

Man erhält auch geeignete Katalysatoren, wenn der isolierte Alumino- bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden können.

Liegen die Zeolithe aufgrund der Art der Herstellung nicht in der katalytisch aktiven, aciden H-Form vor, sondern z.B. in der Na-Form, dann kann diese durch Ionenaustausch, z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 500 °C, insbesondere 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, kann es vorteilhaft sein, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle, wie Li, Cs, K, Erdalkalimetalle, wie Mg, Ca, Sr, Metalle der 3. und 4. und 5. Hauptgruppe, wie Al, Ga, Ge, Sn, Pb, Bi, Übergangsmetalle der 4. - 8. Nebengruppe, wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe, wie Cu, Ag, Zn, seltene Erdmetalle, wie La, Ce, Pr, Nd, Fr, Yb und U eingesetzt.

Zweckmäßigerweise führt man die Dotierung so durch, daß man den verformten Zeolithen in einem Steigrohr vorlegt und bei 20 bis 100 °C eine wäßrige oder ammoniakalische Lösung eines Halogenids oder eines Nitrats der beschriebenen Metalle überleitet. Ein derartiger Ionenaustausch kann an der Wasserstoff-, Ammonium- und Alkaliform des Zeolithen vorgenommen werden. Man kann auch das zeolithische Material mit einem Halogenid, Nitrat oder Oxid der beschriebenen Metalle in wäßriger, alkoholischer oder ammoniakalischer Lösung imprägnieren. Sowohl an einen Ionenaustausch als auch an eine Imprägnierung schließt sich zumindest eine Trocknung, wahlweise eine abermalige Calcinierung an.

Eine mögliche Ausführungsform besteht darin, daß man $Cu(NO_3)_2$ x 3 $H_2O$ oder $Ni(NO_3)_2$ x 6 $H_2O$ oder $Ce(NO_3)_3$ x 6 $H_2O$ oder $La(NO_3)_3$ x 6 $H_2O$ oder $Cs_2CO_3$ in Wasser löst. Mit dieser Lösung wird der verformte oder unverformte Zeolith eine gewisse Zeit, etwa 30 Minuten, getränkt. Die überstehende Lösung wird am Rotationsverdampfer von Wasser befreit. Danach wird der getränkte Zeolith bei etwa 150 °C getrocknet und bei etwa 550 °C calciniert. Dieser Tränkvorgang kann mehrmals hintereinander vorgenommen werden, um den gewünschten Metallgehalt einzustellen.

Es ist auch möglich, eine wäßrige $Ni(NO_3)_2$-Lösung oder ammoniakalische $Pd(NO_3)_2$-Lösung herzustellen und darin den reinen pulverförmigen Zeolithen bei 40 bis 100 °C unter Rühren etwa 24 h aufzuschlämmen. Nach Abfiltrieren, Trocknen bei etwa 150 °C und Calcinierung bei etwa 500 °C kann das so gewonnene zeolithische Material mit oder ohne Bindemittel zu Strängen, Pellets oder Wirbelgut weiterverarbeitet werden.

Ein Ionenaustausch des in der H-Form, Ammonium-Form oder Alkali-Form vorliegenden Zeolithen kann so vorgenommen werden, daß man den Zeolithen in Strängen oder Pellets in einer Kolonne vorlegt und darüber eine wäßrige $Ni(NO_3)_2$-Lösung oder ammoniakalische $Pd(NO_3)_2$-Lösung bei leicht erhöhter Temperatur zwischen 30 und 80 °C im Kreislauf 15 bis 20 h leitet. Danach wird mit Wasser ausgewaschen, bei etwa 150 °C getrocknet und bei etwa 550 °C calciniert. Bei manchen metalldotierten Zeolithen, z.B., Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren, wie Salzsäure, Flußsäure und Phosphorsäure und/oder Wasserdampf unterwirft. Dabei geht man vorteilhaft so vor, daß man die Zeolithe in Pulverform mit 1 n Phosphorsäure 1 Stunde bei 80 °C behandelt. Nach der Behandlung wird mit Wasser gewaschen, bei 110 °C/16 Stunden getrocknet und bei 500 °C/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80 °C mit einer 3 bis 25 Gew.%-igen, insbesondere 12 bis 20 Gew.%-igen wäßrigen Salzsäure. Anschließend wird der so behandelte Zeolith mit Wasser gewaschen, getrocknet und bei 400 °C bis 500 °C calciniert.

Eine besondere Ausführungsform für die Säurebehandlung besteht darin, daß man das zeolitische Material vor seiner Verformung bei erhöhter Temperatur mit 0,001 n bis 2 n, insbesondere 0,05 n bis 0,5 n Flußsäure durch Erhitzen unter Rückfluß über einen Zeitraum von 0,5 bis 5, insbesondere 1 bis 3 Stunden behandelt. Nach Isolierung, durch Abfiltrieren und Auswaschen des zeolithischen Materials wird dieses zweckmäßig bei Temperaturen von 100 bis 160 °C getrocknet und bei Temperaturen von 450 °C bis 600 °C calciniert. Gemäß einer weiteren bevorzugten Ausführungsform für die Säurebehandlung wird das zeolithische Material nach einer Verformung mit Bindemittel bei erhöhter Temperatur, zweckmäßig bei Temperaturen von 50 °C bis 90 °C, insbesondere 60 °C bis 80 °C, über einen Zeitraum von 0,5 bis 5, insbesondere mit 12 bis 20 Gew.%-iger Salzsäure, behandelt. Anschließend wird das zeolithische Material ausgewaschen und zweckmäßig bei Temperaturen von 100 bis 160 °C getrocknet und bei Temperaturen von 450 bis 600 °C calciniert. Einer HF-Behandlung kann sich auch eine HCl-Behandlung anschließen.

Nach einer anderen Arbeitsweise kann man Zeolithe durch Aufbringung von Phosphorverbindungen, wie Trimethoxiphosphat, Trimethoxiphosphin, primärem, sekundärem oder tertiärem Natriumphosphat modifizieren. Hierbei werden die Zeolithe in Strang-, Tabletten- oder Wirbelgut-Form mit wäßriger $H_3PO_4$-Lösung getränkt, bei 110 °C getrocknet und bei 500 °C calciniert.

Weitere Katalysatoren für das erfindungsgemäße Verfahren sind Phosphate, insbesondere Aluminiumphosphate, Siliciumaluminiumphosphate, Siliciumeisenaluminiumphosphate, Cerphosphat, Zirkonphosphate, Borphosphat, Eisenphosphat oder deren Gemische.

Als Aluminiumphosphat-Katalysatoren werden für das erfindungsgemäße Verfahren insbesondere unter hydrothermalen Bedinungen synthetisierte Aluminiumphosphate eingesetzt, die Zeolithstruktur besitzen.

Die unter hydrothermalen Bedingungen hergestellten Aluminiumphosphate sind z.B. APO-5, APO-9, APO-11, APO-12, APO-14, APO-21, APO-25, APO-31 und APO-33. Synthesen dieser Verbindungen sind in EP 132 708, US 4 310 440 und US 4 473 663 beschrieben.

Beispielsweise das $AlPO_4$-5 (APO-5) wird synthetisiert, indem man Orthophosphorsäure mit Pseudoboehmit (Catapal SB®) in Wasser homogen mischt; zu dieser Mischung Tetrapropylammoniumhydroxid gibt und danach bei etwa 150 °C 20 bis 60 h unter autogenem Druck in einem Autoklaven umsetzt. Das abfiltrierte $AlPO_4$ wird bei 100 bis 160 °C getrocknet und bei 450 bis 550 °C calciniert.

$AlPO_4$-9 (APO-9) wird ebenfalls aus Orthophosphorsäure und Pseudoboehmit, aber in wäßriger DABCO-Lösung (1,4-Diazabicyclo-(2,2,2)octan) bei ca. 200 °C unter autogenem Druck während 200 bis 400 h synthetisiert.

Die Synthese des $AlPO_4$-21 (APO-21) erfolgt aus Orthophosphorsäure und Pseudoboehmit in wäßriger Pyrrolidon-Lösung bei 150 bis 200 °C unter autogenem Druck während 50 bis 200 h.

Für das erfindungsgemäße Verfahren geeignete Siliciumaluminiumphosphate sind z.B. SAPO-5, SAPO-11, SAPO-31 und SAPO-34. Die Synthese dieser Verbindung wird in EP-PS 103 117, US-PS 4 440 871 beschrieben. SAPO's werden hergestellt durch Kristallisation aus wäßriger Mischung bei 100 bis 250 °C unter autogenem Druck während 2 h bis 2 Wochen, wobei die Reaktionsmischung aus einer Silicium-, Aluminium- und Phosphorkomponente in wäßrigen aminoorganischen Lösungen umgesetzt wird.

SAPO-5 beispielsweise wird durch Mischen von $SiO_2$ suspendiert in wäßriger Tetrapropylammoniumhydroxid-Lösung mit einer wäßrigen Suspension aus Pseudoboehmit und Orthophosphorsäure und anschließende Umsetzung bei 150 bis 200 °C während 20 bis 200 h unter autogenem Druck in einem Rührautoklaven erhalten. Die Trocknung des abfiltrierten Pulvers erfolgt bei 110 bis 160 °C und die Calcination bei 450 bis 550 °C .

Als Phosphatkatalysatoren kann man bei dem Verfahren auch gefällte Aluminiumphosphate einsetzen. Beispielsweise wird ein derartiges Aluminiumphosphat hergestellt, indem 92 g Diammoniumhydrogenphosphat in 700 ml Wasser gelöst werden. Zu dieser Lösung wird 260 g $Al(NO_3)_3$ x $H_2O$ in 700 ml Wasser über 2 h zugetropft. Dabei wird der pH-Wert durch gleichzeitige Zugabe von 25%iger $NH_3$-Lösung bei pH 8 gehalten. Der entstandene Niederschlag wird 12 h nachgerührt, dann abgesaugt und ausgewaschen. Er wird bei 60 °C /16 h getrocknet.

Borphosphate für das erfindungsgemäße Verfahren lassen sich beispielsweise durch Mischen und Kneten von konzentrierter Borsäure und Phosphorsäure und durch anschließende Trocknung und Calcination in Inertgas-, Luft- oder Dampfatmosphäre bei 250 bis 650 °C, insbesondere 300 bis 500 °C herstellen.

Auf diese Phosphate können durch Imprägnierung (Tränken und Aufsprühen) oder in manchen Fällen auch durch Ionenaustausch Modifizierungskomponenten, wie voran bei den Zeolithen beschrieben, aufgebracht werden. Auch kann wie bei den Zeolithkatalysatoren eine Modifizierung mit Säuren erfolgen.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Die erfindungsgemäße Umsetzung wird vorzugsweise in der Gasphase bei 100 bis 500 °C, insbesondere 200 bis 400 °C, und einer Belastung WHSV = 0,1 bis 20 $h^{-1}$, insbesondere 0,5 bis 5 $h^{-1}$ (g Ausgangsstoff/g Katalysator und Stunde) ausgeführt. Die Reaktion kann in einem Festbett oder auch im Wirbelbett ausgeführt werden.

Es ist auch möglich, die Reaktion in der Flüssigphase (Suspension-, Riesel- oder Sumpffahrweise) bei Temperaturen zwischen 50 und 200 °C durchzuführen.

Das Verfahren kann bei Normaldruck, bei vermindertem oder erhöhtem Druck diskontinuierlich, aber vorzugsweise kontinuierlich durchgeführt werden.

Schwerflüchtige oder feste Ausgangsstoffe werden in gelöster Form, z.B. in THF-, Toluol- oder Petrolether-Lösung eingesetzt. Auch allgemein ist eine Verdünnung der Ausgangsstoffe mit Lösungsmitteln oder mit Inertgasen, wie $N_2$, Ar, $H_2O$-Dampf möglich. In besonderen Fällen kann man auch $O_2$ verwenden.

Nach der Umsetzung werden die Produkte durch übliche Verfahren, z.B. durch Destillation aus dem Reaktionsgemisch isoliert; nichtumgesetzte Ausgangsstoffe werden gegebenenfalls in die Umsetzung zurückgeführt.

Es ist zweckmäßig, die gasförmigen Reaktionsprodukte sofort in die Trennung einzubringen und in die Einzelkomponenten zu zerlegen. Die Trennung kann z.B. in einer Fraktionierkolonne durchgeführt werden.

Beispiele 1-19

Die Reaktionen in der Gasphase werden unter isothermen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgetrennt und charakterisiert. Die quantitative Bestimmung der Reaktionsprodukte und der Ausgangssprodukte erfolgt gaschromatographisch.

Die für das erfindungsgemäße Verfahren eingesetzte Katalysatoren sind:

Katalysator A

Der Borosilikatzeolith des Pentasil-Typs wird in einer hydrothermalen Synthese aus 640 g hochdispersem $SiO_2$, 122 g $H_3BO_3$, 8 000 g einer wäßrigen 1,6-Hexandiamin-Lösung (Mischung 50:50 Gew.% ) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzeolith setzt sich zusammen aus 94,2 Gew.% $SiO_2$ und 2,3 Gew.% $B_2O_3$.

Mit diesem Material werden durch Verformen mit einem Verformungsmittel 2-mm-Stränge hergestellt, die bei 110 °C/16 h getrocknet und bei 500 °C/24 h calciniert werden.

Katalysator B

Katalysator B wird erhalten, indem man den Borosilikatzeolith von Katalysator A mit Boehmit im Gewichtsverhältnis 60:40 zu 2-mm-Strängen verformt, bei 110 °C/16 h trocknet und bei 500 °C/24 h calciniert.

Katalysator C

Ein Aluminosilikatzeolith vom Pentasil-Typ wird unter hydrothermalen Bedingungen bei autogenem Druck und 150 °C aus 65 g hochdispersem $SiO_2$, 20,3 g $Al_2(SO_4)_3$ x 18 $H_2O$ in 1 kg einer wäßrigen 1,6-Hexandiamin-Lösung (Mischung 50:50 Gew.% ) in einem Rührautoklaven hergestellt. Nach Abfiltrieren und Auswaschen wird das kristalline Reaktionsprodukt bei 110 °C/24 h getrocknet und bei 500 °C/24 h calciniert. Dieser Aluminosilikatzeolith enthält 91,6 Gew.% $SiO_2$ und 4,6 Gew.% $Al_2O_3$.

Der Katalysator wird mit einem Verformungshilfsmittel zu 2-mm-Strängen verformt, bei 110 °C/16 h getrocknet und bei 500 °C/24 h calciniert.

Katalysator D

Katalysator E wird erhalten, indem man die Stränge des Katalysators B mit einer wäßrigen Lösung aus Cernitrat imprägniert, danach bei 130 °C/2 h trocknet und bei 540 °C/2 h calciniert. Der Ce-Gehalt beträgt 2,5 Gew.%.

Katalysator E

Der Eisensilikatzeolith des Pentasil-Typs wird unter hydrothermalen Bedingungen bei autogenem Druck und 165 °C aus 273 g Wasserglas, gelöst in 253 g einer wäßrigen 1,6-Hexandiamin-Lösung (Mischung 50:50 Gew.%) und 31 g Eisensulfat, gelöst in 21 g 96%iger Schwefelsäure und 425 g Wasser in einem Rührautoklaven während 4 Tagen synthetisiert. Der Zeolith wird abfiltriert, ausgewaschen, bei 110 °C/24 h getrocknet und bei 500 °C/24 h calciniert. Man erhält einen Eisensilikatzeolithen mit einem $SiO_2/Fe_2O_3$-Verhältnis von 17,7 und einen $Na_2O$-Gehalt von 1,2 Gew.%. Der Katalysator wird mit hochdispersem $SiO_2$im Gewichtsverhältnis 80:20 zu 2,5-mm-Strängen verstrangt, brei 110 °C/16 h getrocknet und bei 500 °C/24 h calciniert.

Katalysator F

Siliciumaluminiumphosphat-5 (SAPO-5) wird aus einer Mischung aus 200 g 98%ige Phosphorsäure, 136 g Boehmit, 60 g Kieselsol (30%ig), 287 g Tripropylamin und 587 g $H_2O$ hergestellt. Diese Mischung wird

bei 150 °C während 168 h unter autogenem Druck umgesetzt. Nach Filtration wird das kristalline Produkt bei 120 °C getrocknet und bei 500 °C calciniert. SAPO-5 enthält 49,8 Gew.% $P_2O_5$, 33,0 Gew.% $Al_2O_3$, 6,2 Gew.% $SiO_2$. SAPO-5 wird mit einem Verstrangungshilfsmittel zu 3-mm-Strängen verformt, bei 120 °C getrocknet und bei 500 °C calciniert.

Katalysator G

Im Handel erhältliches Zirkonphosphat $Zr_3(PO_4)_4$ wird in Reinsubstanz verformt.

Katalysator H

$BPO_4$ wird hergestellt, indem man 49 g $H_3BO_3$ mit 117 g $H_3PO_4$ /75%ig) in einem Kneter zusammengibt, überschüssiges Wasser abdampft und das Umsetzungsprodukt zu 3-mm-Strängen verformt. Diese Stränge werden bei 100 °C getrocknet und bei 350 °C calciniert. Katalysator N enthält 8,77 Gew.% B und 28,3 Gew.% P.

Die mit diesen Katalysatoren erzielten Versuchsergebnisse und die Versuchsbedingungen sind in Tabelle 1 zusammengefaßt.

Man erkennt, daß die zeolithischen Katalysatoren unter allen genannten Katalysatoren am besten für das erfindungsgemäße Verfahren geeignet sind.

Tabelle

| Beispiel | 1 | 2 | 3 | 4 | 5 | 6 | 7 | 8 | 9 |
|---|---|---|---|---|---|---|---|---|---|
| Einsatzstoff | | 1,4-Butandiformiat | | 1-Methyl-1,3-propandiformiat | | | | | |
| Katalysator | A | C | C | A | A | B | C | D | E |
| Temperatur [°C] | 300 | 200 | 300 | 200 | 300 | 300 | 300 | 300 | 300 |
| WHSV $h^{-1}$ | 4 | 4,5 | 3 | 2,5 | 4 | 4 | 3 | 4 | 4 |
| Umsatz [%] | 34,6 | 21,5 | 95,0 | 12,0 | 50,6 | 59,3 | 65,4 | 61,2 | 44,1 |
| Selektivität [%] | | | | | | | | | |
| ungesättigter Ester | 83,8 | 65,1 | 93,3 | 66,7 | 89,9 | 88,5 | 82,7 | 90,5 | 77,8 |

Neben- bzw. Koppelprodukte sind z.B. Butadien, CO, $CO_2$, $H_2O$, Ameisensäure, Essigsäure, Aceton

Tabelle

| Beispiel | 10 | 11 | 12 | 13 | 14 | 15 | 16 | 17 | 18 | 19 |
|---|---|---|---|---|---|---|---|---|---|---|
| Einsatzstoff | 1,6-Hexandiolacetat | | | | | | | 1,4-Butandiacetat | | |
| Katalysator | A | C | C | D | F | G | H | A | E | E |
| Temperatur [°C] | 300 | 300 | 400 | 300 | 300 | 300 | 300 | 400 | 300 | 400 |
| WHSV $h^{-1}$ | 4 | 2 | 4 | 4 | 2 | 2 | 2 | 3 | 3 | 3 |
| Umsatz [%] | 22,9 | 58,9 | 77,8 | 49,4 | 33,2 | 42,7 | 27,9 | 71,0 | 16,8 | 100 |
| Selektivität [%] | | | | | | | | | | |
| ungesättigter Ester | 86,9 | 79,5 | 78,3 | 69,4 | 78,1 | 76,3 | 81,2 | 83,9 | 96,4 | 80,8 |

Neben- bzw. Koppelprodukte sind z.B. Butadien, CO, $CO_2$, $H_2O$, Ameisensäure, Essigsäure, Aceton

## Patentansprüche

1. Verfahren zur Herstellung ungesättigter Monoester der Formel (I)

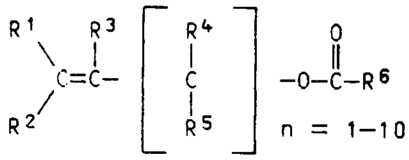

(I)

dadurch gekennzeichnet, daß man Diester der Formel II

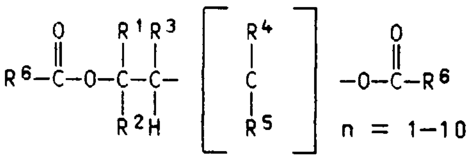

(II)

wobei die Reste $R^1$ bis $R^5$ in den Formeln (I) und (II) einander gleich oder verschieden sind und für Wasserstoff, geradkettige oder verzweigte Alkyl- oder Alkenylreste mit 1 bis 8 Kohlenstoffatomen, Cycloalkyl- oder Cycloalkenylreste mit 5 bis 8 Kohlenstoffatomen, Aryl-, Alkylaryl- oder Aralkylreste stehen, unter Abspaltung von Carbonsäuren $R^6COOH$, wobei $R^6$ für Wasserstoff oder Alkylrest mit 1 bis 6 Kohlenstoffatomen steht, in Gegenwart von Zeolithen und/oder Phosphaten als Katalysatoren umsetzt.

2. Verfahren nach Anspruch 1, dadurch gekennzeichnet, daß man Butandiacetat, Hexandiacetat und Octandiacetat umsetzt.

3. Verfahren nach Anspruch 1 und 2, dadurch gekennzeichnet, daß man als Katalysatoren Aluminiumsilikatzeolithe und/oder Eisensilikatzeolithe des Pentasiltyps verwendet.

4. Verfahren nach Anspruch 1 und 2, dadurch gekennzeichnet, daß man als Katalysatoren Zeolithe des Faujasittyps verwendet.

5. Verfahren nach Anspruch 1 bis 4, dadurch gekennzeichnet, daß man mit Alkalimetallen, Übergangsmetallen, seltenen Erdmetallen dotierte Zeolithe als Katalysatoren verwendet.

6. Verfahren nach Anspruch 1, dadurch gekennzeichnet, daß man als Katalysatoren Phosphate der Elemente B, Al, Zr, Ce, Fe, Sr oder deren Gemische verwendet.

7. Verfahren nach Anspruch 1, 2 und 6, dadurch gekennzeichnet, daß man als Katalysatoren hydrothermal hergestellte Phosphate, wie Aluminiumphosphate, Siliciumaluminiumphosphate, Siliciumeisenaluminiumphosphate oder Boraluminiumphosphate verwendet.

8. Verfahren nach Anspruch 1 bis 7, dadurch gekennzeichnet, daß man die Reaktion in der Gasphase durchführt.

## Claims

1. A process for preparing an unsaturated monoester of the formula (I)

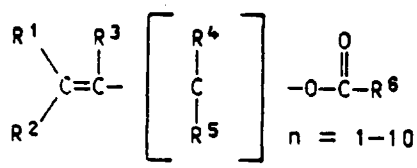

(I)

by reacting a diester of the formula II

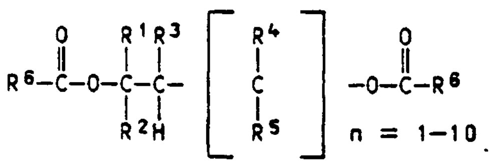

(II)

where the radicals $R^1$ to $R^5$ in the formulae (I) and (II) are identical to each other or different from each other and each is hydrogen, straight-chain or branched alkyl or alkenyl of from 1 to 8 carbon atoms, cycloalkyl or cycloalkenyl of from 5 to 8 carbon atoms, aryl, alkylaryl or aralkyl, in the presence of a zeolite or phosphate as a catalyst with the elimination of a carboxylic acid $R^6COOH$ where $R^6$ is hydrogen or alkyl of from 1 to 6 carbon atoms.

2. A process as claimed in claim 1, wherein an acetoxybutyl acetate, an acetoxyhexyl acetate or an acetoxyoctyl acetate is converted.

3. A process as claimed in claim 1 or 2, wherein the catalyst used is an aluminosilicate zeolite or an iron silicate zeolite of the pentasil type.

4. A process as claimed in claim 1 or 2, wherein the catalyst used is a zeolite of the faujasite type.

5. A process as claimed in any of claims 1 to 4, wherein a zeolite doped with an alkali metal, a transition metal or a rare earth metal is used as the catalyst.

6. A process as claimed in claim 1, wherein the catalyst used is a phosphate of the element B, Al, Zr, Ce, Fe or Sr or a mixture thereof.

7. A process as claimed in claim 1, 2 or 6, wherein the catalyst used is a hydrothermally synthesized phosphate, such as aluminum phosphate, silicon aluminum phosphate, silicon iron aluminum phosphate or boron aluminum phosphate.

8. A process as claimed in any of claims 1 to 7, wherein the reaction is carried out in the gas phase.

## Revendications

1. Procédé de préparation de monoesters insaturés de formule (I)

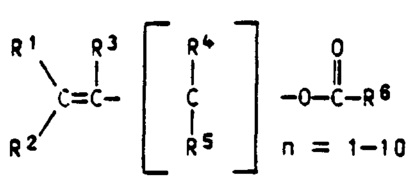

(I)

caractérisé en ce qu'on fait réagir, en présence de zéolithes et/ou de phosphates servant de

catalyseurs, des diesters de formule II

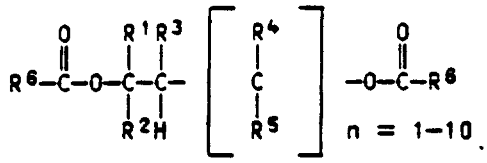

(II)

les restes $R^1$ à $R^5$ dans les formules (I) et (II) étant identiques au différents les uns des autres et étant mis pour des atomes d'hydrogène, pour des restes alkyle ou alcényle à chaîne droite ou ramifiée à 1-8 atomes de carbone, pour des restes cycloalkyle ou cycloalcényle à 5-8 atomes de carbone ou pour des restes aryle, alkylaryle ou aralkyle, avec élimination d'acides carboxyliques $R^6COOH$, $R^6$ étant mis pour un atome d'hydrogène ou un reste alkyle à 1-6 atomes de carbone.

**2.** Procédé selon la revendication 1, caractérisé en ce qu'on fait réagir du butanediacétate, de l'hexanediacétate et de l'octanediacétate.

**3.** Procédé selon la revendication 1 ou 2, caractérisé en ce qu'on utilise, comme catalyseurs, des zéolithes d'aluminosilicate et/ou des zéolithes de ferrosilicate du type pentasil.

**4.** Procédé selon la revendication 1 ou 2, caractérisé en ce qu'on utilise, comme catalyseurs, des zéolithes du type faujasite.

**5.** Procédé selon l'une quelconque des revendications 1 à 4, caractérisé en ce qu'on utilise, comme catalyseurs, des zéolithes dopées avec des métaux alcalins, des métaux de transition ou des métaux des terres rares.

**6.** Procédé selon la revendication 1, caractérisé en ce qu'on utilise, comme catalyseurs, des phosphates des éléments B, Al, Zr, Ce, Fe, Sr ou des mélanges de ceux-ci.

**7.** Procédé selon les revendications 1, 2 et 6, caractérisé en ce qu'on utilise, comme catalyseurs, des phosphates préparés par synthèse hydrothermale, tels que des phosphates d'aluminium, des phosphates de silicium et d'aluminium, des phosphates de silicium, de fer et d'aluminium ou des phosphates de bore et d'aluminium.

**8.** Procédé selon l'une quelconque des revendications 1 à 7, caractérisé en ce qu'on conduit la réaction en phase gazeuse.